# FASCICULE DE BREVET EUROPEEN

(11) **EP 1 651 303 B1**
(45) Date de publication et mention de la délivrance du brevet: **22.10.2008**
(21) Numéro de dépôt: 04767795.0
(22) Date de dépôt: 28.07.2004
(51) Int. Cl.: A61M 25/06, A61M 29/00, A61B 1/00

(54) **DISPOSITIF D`AIDE A LA POSE PERCUTANEE D`UN TUBE GUIDE POUR UN NEPHROSCOPE DANS LA CHIRURGIE DU REIN.**
VORRICHTUNG ZUR PERKUTANEN VERLEGUNG EINER FÜHRUNGSRÖHRE FÜR EIN NEPHROSKOP IN DER NIERENCHIRURGIE
DEVICE FOR AIDING THE PERCUTANEOUS POSITIONING OF A GUIDING TUBE FOR A NEPHROSCOPE IN KIDNEY SURGERY

(30) Priorité: 01.08.2003 FR 0309481
(43) Date de publication de la demande: 03.05.2006
(73) Titulaire: LMA Urology Limited, Victoria, Mahe (SC)
(72) Inventeur: LEBET, Alain, 1006 Lausanne (CH)
(74) Mandataire: Jacob, Reuben Ellis
(86) Numéro de dépôt international: PCT/FR2004/002013
(87) Numéro de publication internationale: WO 2005/014096

(56) Documents cités:
- DE-A- 3 206 381
- US-A- 5 707 382
- US-A- 5 810 790

## Description

L'invention concerne un dispositif d'aide à la pose percutanée d'un tube guide pour un néphroscope dans la chirurgie du rein.

Voir par exemple le document US-A-5 707 382.

Le tube guide à poser est une gaine en matière plastique, semi translucide à la radio, de l'ordre de dix millimètres de diamètre comportant un épaulement du côté de l'extrémité extérieure ; la pose d'un tel tube guide, aussi appelé « sheat », se fait actuellement de plusieurs façons ; cela consiste d'abord à enfoncer une aiguille de ponction d'un diamètre de treize à vingt gauges depuis la surface de la peau jusque dans le rein ; cette aiguille de ponction est une aiguille creuse qui est obturée sur toute sa longueur par un mandrin pendant la phase d'enfoncement de l'aiguille de ponction dans le rein puis lorsque la profondeur d'enfoncement permettant d'atteindre le bassinet du rein est atteinte, le mandrin est enlevé pour vérifier qu'il se produit un écoulement d'urine indiquant que l'extrémité de l'aiguille de ponction est bien dans le bassinet du rein ; d'une part un guide métallique souple est alors enfoncé dans l'aiguille de ponction qui est alors retirée et d'autre part un premier tube dilatateur, appelé « tube dilatateur d'Alken » est enfilé sur le guide métallique par coulissement jusqu'à ce que son extrémité distrale vienne buter sur l'extrémité du guide métallique souple qui s'est enroulée en boule ; le diamètre intérieur du premier tube dilatateur est de l'ordre de treize à vingt gauges et son épaisseur est de l'ordre de un tiers de millimètres soit un « franche » qui est une unité de mesure utilisée en technologie chirurgicale ; le premier tube dilatateur comporte un épaulement à son extrémité distale qui sert de butée à une série de tubes dilatateurs gigognes qui s'enfilent les uns au-dessus des autres afin d'augmenter le diamètre de l'ouverture effectuée dans le rein ; chacun des tubes dilatateurs d'Alken augmente le diamètre de l'ouverture de deux « franche » et lorsque son diamètre est suffisant il reste à enfiler le tube guide sur le dernier tube dilatateur ; il suffit ensuite de retirer l'ensemble des tubes dilatateurs ; cette technique nécessite un grand nombre de clichés radiologiques de contrôle.

Une autre technique plus rapide consiste à enfiler sur l'aiguille un tube support métallique comportant un ballonnet gonflable ; ce ballonnet est constitué d'une enveloppe souple, entourant le tube support, qui est très peu élastique et qui, lorsqu'elle est gonflée, est de forme allongée cylindrique de révolution dans sa partie centrale, allant en se rétrécissant aux extrémités jusqu'à atteindre un diamètre intérieur sensiblement égal au diamètre du tube support ; les extrémités du ballonnet étant alors serties sur le tube support à l'aide de tubes de sertissage métalliques ; l'enveloppe constituant le ballonnet comporte une tubulure de gonflage permettant d'y introduire un liquide de gonflage en quantité nécessaire et suffisante pour que la partie centrale atteigne un diamètre sensiblement égal à celui du tube guide à installer ; avant l'opération d'élargissement de l'ouverture du rein, par introduction et gonflage du ballonnet il est nécessaire d'effectuer dans la chair du patient, avec un bistouri, des coupes radiales autour de l'aiguille de manière à éviter que les chairs ne se déchirent au cours du gonflage ce qui rend la cicatrisation plus difficile ; lorsque le diamètre de gonflage est atteint, il suffit de faire glisser le tube guide sur l'enveloppe du ballonnet qui est lubrifiée par un enduit imprégnant la surface du ballonnet et qui devient lubrifiant par simple humidification ; puis, lorsque le tube guide est en place, l'ensemble constitué par le tube de support et l'aiguille de ponction est retirée. L'opération d'élargissement de la plaie est difficile à réaliser manuellement surtout en profondeur et étant en grande partie réalisée sans possibilité de contrôle visuel, elle ne permet pas toujours d'éviter le déchirement des chairs autour de la plaie.

Il existe d'autre part des guides métalliques souples qui sont enfilés dans l'aiguille de ponction et qui restent en place lorsque l'aiguille de ponction est enlevée pour pouvoir être utilisée en cas de problème en cours d'opération ; un guide métallique souple est constitué d'une âme métallique cylindrique entourée d'un fil plastique à spires jointives. Il existe des métaux à mémoire de forme qui sous fourme de fil ont une forme initiale qui peut être modifiée à volonté mais qui revient à la forme initiale dès que certaines conditions sont réunies.

L'objet de l'invention consiste à proposer un dispositif de pose de tube guide, à usage unique, permettant de positionner rapidement un tube guide pour néphroscope.

La description qui suit s'appuie sur les figures suivantes :
La figure 1 représente une vue perspective éclatée d'un dispositif de pose de tube guide selon l'invention.
La figure 2 représente une coupe longitudinale d'un dispositif de pose d'un tube guide selon l'invention assemblé par surmoulage de matière plastique.
La figure 3 représente une coupe transversale de l'assemblage du trocart selon la figure 2.

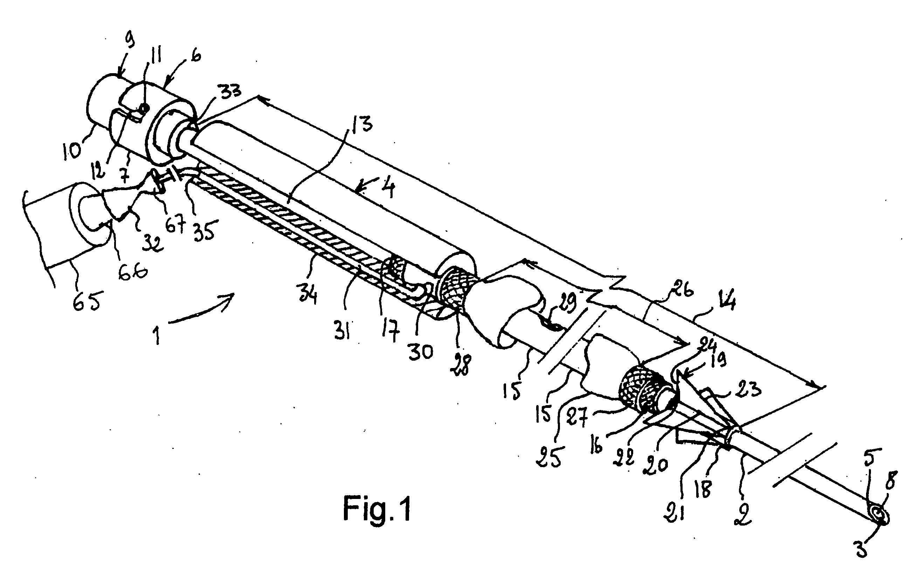

L'invention consiste en un dispositif de pose de tube guide 1 (fig.1) pour néphroscope constitué d'une longue aiguille de ponction 2, munie d'un mandrin d'obturation 3, sur laquelle coulisse un dispositif d'élargissement gonflable 4, plus court que l'aiguille de ponction 2 qui lui sert de moyen de guidage, le dispositif d'élargissement gonflable 4 servant lui-même de moyen de guidage à un tube guide pour néphroscope lors de sa mise en place sur un patient. L'aiguille de ponction 2 a une première extrémité d'aiguille 5 taillée en biseau et une deuxième extrémité d'aiguille 6 comportant un manchon d'aiguille 7, entourant la partie latérale de la deuxième extrémité d'aiguille, sensiblement cylindrique de révolution de préférence coaxialement à l'aiguille de ponction 2, d'un diamètre sensiblement inférieur à celui du tube guide à poser ; l'aiguille de ponction 2 a une longueur sensiblement du double de la longueur nécessaire pour atteindre le bassinet du rein de manière que la moitié de l'aiguille de ponction 2 située du côté de la deuxième extrémité d'aiguille 6 serve de support au dispositif d'élargissement gonflable 4 pendant la phase de l'intervention consistant à introduire la première extrémité d'aiguille 5 dans le bassinet du rein ; l'aiguille de ponction 2 comporte un mandrin d'obturation 3 dont une première extrémité de mandrin 8 est taillée en biseau comme la première extrémité 5 de l'aiguille de ponction 2 et située dans le même plan que celui de la première extrémité d'aiguille 5 tandis que la deuxième extrémité de mandrin 9 comporte un dispositif de fixation et d'orientation 10 du mandrin d'obturation 3 sur le manchon d'aiguille 6 ; le dispositif de fixation et d'orientation 10 est, par exemple, constitué d'un système de fixation 12 dans lequel au moins un téton 11 solidaire de la deuxième extrémité de mandrin 9 vient se clipser ; lorsque le mandrin d'obturation 3 est enlevé, il est remplacé par un guide à mémoire de forme (non représenté sur le dessin) qui est normalement recourbé à son extrémité mais qui peut. être maintenu initialement rectiligne, par exemple, par déformation mécanique le temps de traverser l'aiguille de ponction 2 jusque dans le rein ; dès que l'extrémité du guide à mémoire de forme sort, au niveau de la première extrémité d'aiguille 5, il reprend sa forme pour servir de butée de positionnement au dispositif d'élargissement gonflable 4 ; le mandrin d'obturation 3 peut remplacer avantageusement la pose d'un guide en le réalisant avec un métal à mémoire de forme et en utilisant un manchon d'aiguille 7 en deux parties dont une partie servant d'appui à la deuxième extrémité de mandrin 9 est amovible et permet le dépassement de la première extrémité de mandrin 8 de la première extrémité d'aiguille 5 et la formation d'un coude de retenue.

Le dispositif d'élargissement gonflable 4 est composé d'un tube de coulissement 13, qui est métallique, dont le diamètre intérieur est légèrement supérieur à celui de l'aiguille de ponction 2 et dont la longueur 14 est de préférence inférieure à la moitié de la longueur de l'aiguille de ponction 2 ; ce tube de coulissement 13 est susceptible de coulisser tout le long de l'aiguille de ponction 2 et d'en échapper par la première extrémité d'aiguille 5 ; un tube support 15 plus court que le tube de coulissement 13 vient entourer le tube de coulissement 13 auquel il est fixé par ses extrémités 16 et 17 de manière étanche ; le diamètre intérieur du tube support 15 est plus grand que le diamètre extérieur du tube de coulissement 13 d'une valeur nécessaire et suffisante pour permettre le passage d'un liquide de gonflage sous pression ; l'étanchéité entre le tube de coulissement 13 et le tube support 15 est obtenue, par exemple, par rétreint ou par positionnement d'un manchon de centrage (non représenté sur le dessin) entre le tube de coulissement 13 et le tube support 15 suivi, par exemple, d'une étanchéification par introduction de soudure par capillarité entre les tubes de coulissement et support 13 et 15 et le manchon de centrage ; le tube de coulissement 13 comporte une première extrémité de tube de coulissement 18 tournée vers la première extrémité d'aiguille 5 qui comporte au moins deux lames coupantes 19, en forme sensiblement de triangle rectangle, placées dans un plan de symétrie passant par l'axe du tube de coulissement 13 et, par exemple, symétriques entre elles par rapport à ce dernier ; un premier côté de lame 20 de chacun des triangles constituant les lames coupantes 19 est solidaire du tube de coulissement 13 auquel il est fixé suivant une génératrice du tube de coulissement 13 ; une manière de fixer les lames coupantes 19 consiste à les souder sur le tube de coulissement 13, par exemple, en utilisant une soudure au laser ; une première extrémité de premier côté de lame 21 coïncide sensiblement avec la première extrémité de tube de coulissement 18 située du côté de la première extrémité d'aiguille 5 ; une deuxième extrémité de premier côté 22 est située au voisinage immédiat d'une première extrémité de tube support 16 ; un deuxième côté de lame 23 de chaque lame coupante 19 comporte un fil coupant ; une première extrémité de deuxième côté de lame est confondue avec la première extrémité de premier côté 21 ; le premier côté de lame 20 formant un angle de l'ordre de cinq à dix degrés avec le deuxième côté de lame 23 ; le deuxième côté de lame 23 est de préférence profilé pour permettre une meilleure efficacité de la coupure effectuée par le fil coupant, par exemple, en lui donnant une forme concave ; un troisième côté de lame 24, qui est non coupant, est sensiblement perpendiculaire au premier côté de lame 20 et ayant une longueur telle que le cumul du diamètre du tube de coulissement 13 et de la longueur des deux troisièmes côtés de lames 24 symétriques soit de l'ordre de grandeur du diamètre interne du tube guide à poser. Dans une version préférée de l'invention, les lames coupantes 19 sont au nombre de quatre à six ; lorsque, par exemple, elles sont au nombre de quatre, elles sont symétriques entre elles deux à deux suivant deux plans de symétrie perpendiculaires entre eux et passant par l'axe de symétrie du tube de coulissement 13 ; l'angle entre le deuxième et le troisième côté 23 et 24 de lame est remplacé par un arrondi ; cet ensemble de lames coupantes 19 est appelé un «trocart». Un ballonnet 25, d'une longueur 26 sensiblement égale à celle des ballonnets déjà utilisés, recouvre le tube support 15 sur lequel il est fixé à ses extrémités 27 et 28, par exemple, par des tubes de sertissage comme précédemment décrit ; une première extrémité de ballonnet 27 est à proximité immédiate des troisièmes côtés de lame 24 et de la première extrémité de tube support 16 ; la deuxième extrémité de ballonnet 28 est fixée sur le tube support 15 qui est très sensiblement plus long que le ballonnet 25 ; le tube support 13 est percé, d'une part d'un premier orifice 29 dans la zone située à l'intérieur du ballonnet 25 et, d'autre part, d'un deuxième orifice 30 situé au voisinage d'une deuxième extrémité de tube support 17 qui est extérieure au ballonnet 25 ; le deuxième orifice 30 est relié à une tubulure de remplissage 31, munie d'un dispositif d'obturation 67, susceptible de communiquer avec un dispositif d'alimentation 65 en liquide de gonflage sous pression, par exemple, par l'intermédiaire d'un dispositif de raccordement composé d'un premier dispositif interface de raccordement 32 solidaire de la tubulure de remplissage 31 et d'un deuxième dispositif interface de raccordement 66 solidaire du dispositif d'alimentation 65 en liquide de gonflage sous pression ; un dispositif d'alimentation 65 de liquide de gonflage sous pression est, par exemple, une seringue composée d'un réservoir cylindrique dans lequel coulisse un piston dont la tige de commande est filetée et vissée dans un écrou solidaire du réservoir ; en comptant le nombre de tours de la tige de commande dans le sens du vissage, il est possible d'en déduire exactement le volume de liquide de gonflage délivré par le dispositif d'alimentation 65 ; le deuxième dispositif interface de raccordement 66 est constitué, par exemple, d'un cône mâle venant s'emboîter dans un cône femelle constituant le premier dispositif interface de raccordement 32 ; le liquide de gonflage est, par exemple, du sérum physiologique ; le dispositif d'obturation 67 est, par exemple, un dispositif à bille dont la bille est appuyée sur un siège conique par un ressort et qui laisse passer le liquide de gonflage du dispositif d'alimentation 65 vers le ballonnet 25 mais empêche le liquide de gonflage de revenir ; la surface extérieure du ballonnet 25 est enduite d'une substance qui devient gluante au contact de l'eau et qui permet de lubrifier le glissement du tube guide sur le ballonnet ; la tubulure de remplissage peut être métallique et soudée au tube support 15 au niveau du deuxième orifice 30 ; pour permettre le passage du tube guide, l'ensemble constitué du tube support 15 du tube de coulissement 13, de la tubulure de remplissage 31 munie de son premier dispositif interface de raccordement 32, doit s'inscrire dans un volume cylindrique de révolution virtuel dont l'axe de symétrie est celui du tube de coulissement 13 et dont le diamètre est inférieur au diamètre interne du tube guide ; enfin la partie du tube de coulissement 13 située au voisinage de la deuxième extrémité de tube de coulissement 33, éventuellement la partie du tube support 15 et de la tubulure de remplissage 31 situées près de la deuxième extrémité de tube support 17 sont recouvertes d'un manche de manipulation 34, inscrit dans le volume cylindrique de révolution virtuel précédemment défini, dont la deuxième extrémité, située du côté de la deuxième extrémité de tube de coulissement 33, est positionnée par rapport à cette dernière de telle sorte que le tube de coulissement 13 ne puisse gêner le raccordement du dispositif d'alimentation 65.

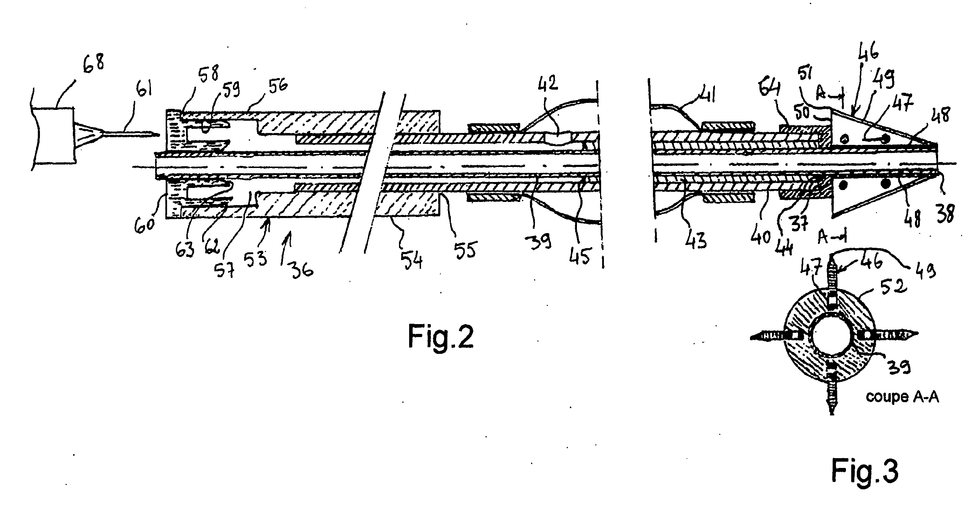

Une manière d'industrialiser la fabrication d'un dispositif d'aide à la pose de tube guide 36 (fig.2), consiste, par exemple, à utiliser la matière plastique pour assembler entre elles, les principales pièces du dispositif d'aide à la pose de tube guide 36 par la technique du surmoulage.

Un premier surmoulage concerne la zone située du côté des premières extrémités 37 et 38 du tube de coulissement 39 et du tube support 40 ; le tube support 40 comporte au moment de sa mise en oeuvre un ballonnet 41 communiquant avec l'intérieur du tube support 40 par le premier orifice 42 ; le tube support 40 est positionné par rapport au tube de coulissement 29 à l'aide d'un manchon de centrage 43 dont la première extrémité de manchon de centrage 44 commence, par exemple, au niveau de la première extrémité de tube support 37 et dont la longueur est telle que la deuxième extrémité de tube de centrage 45 n'obture pas le premier orifice 42 ; l'ensemble constitué par le tube de coulissement 39, le tube support 40 et le manchon de centrage 43 est introduit par la première extrémité de tube de coulissement 38 dans un logement de première extrémité (non représenté sur le dessin) approprié d'un moule d'injection ; ce logement de première extrémité comporte notamment un noyau qui s'introduit et s'ajuste à l'intérieur du tube de coulissement pour éviter que la matière y pénètre ; les lames coupantes 46, qui comportent des perçages de lames 47 le long du premier côté de lame 48, sont positionnées chacune dans un logement du moule d'injection (non représenté sur le dessin) qui protège le deuxième côté de lame 49 qui est la partie coupante et la partie du troisième côté de lame 50 située du côté de la deuxième extrémité du deuxième côté de lame 51 ; lorsque la matière thermoplastique est injectée elle forme, par exemple, une sorte de tronc de cône de révolution 52 (fig.3) enserrant la base des lames coupantes 46 qui sont verrouillées par la matière plastique traversant les perçages de lames 47 ; le tronc de cône de révolution 52 enserrant le tube de coulissement 39 ; le tronc de cône de révolution 52 se prolongeant après les troisièmes côtés de lames 50 (fig.2) par un cylindre d'étanchéification 64 venant recouvrir la première extrémité de tube support 37 sur une longueur suffisante pour assurer l'étanchéité aux liquides de l'espace situé entre le tube de coulissement 39 et le tube support 40 et cela sans gêner le ballonnet 41.

Un deuxième surmoulage permet de réaliser le manche de manipulation 53 (fig.2) dont une première partie de manche 54 massive est située du côté de la première extrémité de manche 55 et tournée du côté du ballonnet 41 recouvre une zone du tube support 40, une deuxième partie de manche 56 étant évidée pour constituer la tubulure de remplissage 57 ; la deuxième extrémité de manche 58 étant constituée d'un goulot de manche 59 ouvert sur l'extérieur et traversé coaxialement par le tube de coulissement 39.

Si la pression nécessaire pour le gonflage du ballonnet 41 peut être relativement importante, la pression résiduelle après gonflage est beaucoup plus faible, en conséquence, un premier dispositif interface de raccordement et d'obturation peut être constitué d'un simple bouchon en caoutchouc 60 qui est introduit dans le goulot de manche 59 pour obturer la tubulure de remplissage 57 et constituer en même temps le premier dispositif interface de raccordement tandis que le deuxième dispositif interface de raccordement est constitué d'une aiguille 61, par exemple, pour piqûre hypodermique, qui permet de passer à travers le bouchon en caoutchouc 60 pour injecter le liquide de gonflage contenu dans un dispositif d'alimentation 68 et constitué, par exemple, par une seringue ; le bouchon en caoutchouc 60 gardant une étanchéité suffisante lorsque l'aiguille 61 est retirée.

Plus particulièrement, il est alors possible de fermer le goulot de manche 59 par le bouchon en caoutchouc 60 comportant une jupe d'étanchéité externe 62 et une jupe d'étanchéité interne 63 ; la jupe d'étanchéité externe 63 assurant l'étanchéité du côté intérieur du goulot de manche 59 et la jupe d'étanchéité interne 63 assurant l'étanchéité avec le côté externe du tube de coulissement 39.

Initialement, l'aiguille de ponction 2 (fig.1) munie de son mandrin 2 est en place dans le dispositif d'élargissement gonflable 4 avec son manchon d'aiguille 7 en butée sur la deuxième extrémité de tube de coulissement 33 de tube de coulissement 13 ; en prenant appui sur le manchon d'aiguille 7, l'aiguille de ponction 2 est d'abord enfoncée dans le rein en traversant la peau du patient jusqu'à ce que son extrémité atteigne le bassinet du rein ; le mandrin d'obturation 3 est alors enlevé permettant de constater que de l'urine s'écoule à travers l'aiguille de ponction 2 et soit le mandrin d'obturation 3 est remis en place après avoir enlevé la partie amovible, du manchon d'aiguille 7, servant d'appui à la deuxième extrémité de mandrin 9, soit un guide à mémoire de forme est mis en place ; le dispositif d'élargissement gonflable 4 est alors enfoncé en coulissant le long de l'aiguille de ponction 2 qui sert de guidage ; le dispositif d'élargissement gonflable 4 est enfoncé dans le rein en s'appuyant sur le manche de manipulation 34 jusqu'à ce que la première extrémité de tube de coulissement 18 arrive à la première extrémité d'aiguille de ponction 5 située dans le bassinet du rein et matérialisée par l'extrémité recourbée du mandrin d'obturation 3 ou du guide à mémoire de forme ; l'aiguille de ponction 2 peut alors être retirée dans le cas de l'utilisation d'un guide à mémoire de forme, mais elle reste en place dans le cas de l'utilisation d'un mandrin d'obturation à mémoire de forme ; le dispositif d'alimentation 65 est raccordé sur la tubulure de remplissage 31 et le ballonnet 25 est gonflé de manière contrôlée ; ensuite le tube guide est mis en place et le dispositif d'élargissement gonflable est retiré en laissant éventuellement en place le guide à mémoire de forme et dans le cas de l'utilisation d'un mandrin d'obturation à mémoire de forme en plaçant au moins un guide à la place du dispositif d'élargissement gonflable 4.

## Revendications

1. Dispositif de pose de tube guide (1) pour néphroscope, utilisant une aiguille de ponction (2), munie d'un manchon d'aiguille (7) et d'un mandrin d'obturation (3), servant de moyen de guidage à un dispositif d'élargissement gonflable (4,36) équipé d'un ballonnet (25,41) rempli par un liquide de gonflage, **caractérisé en ce que** le dispositif d'élargissement gonflable (4,36) est composé, d'un tube de coulissement (13,39) susceptible de coulisser tout le long de l'aiguille de ponction (2), d'un tube support (15,40), autour duquel est fixé le ballonnet (25,41), entourant le tube de coulissement (13,39), dont le diamètre intérieur est inférieur au diamètre intérieur du tube support (15,40) constituant un espace de circulation du liquide de gonflage communiquant par un premier orifice (29,42) avec l'intérieur du ballonnet (25,41) et par un deuxième orifice avec une tubulure de remplissage (31,57), munie d'un dispositif d'obturation (67,60) raccordée à un premier dispositif interface de raccordement (32,60) susceptible de coopérer avec un deuxième dispositif interface de raccordement (66,61) communiquant avec un dispositif d'alimentation (65,68) en liquide de gonflage, le tube support (15,40) étant raccordé par au moins une de ses extrémités (16,17,44) au tube de coulissement (13,39) de manière étanche, une première extrémité de tube de coulissement (18) du tube de coulissement (13) comportant au moins deux lames coupantes (19,46) constituant un trocart et une deuxième extrémité de tube de coulissement (33) comportant un manche de manipulation (34,53).

2. Dispositif selon la revendication 1, **caractérisé en ce que** l'étanchéité entre une première et deuxième extrémité (16,17) du tube support (13) et le tube de coulissement (15) est obtenue par rétreint ou par positionnement d'un manchon de centrage (43) entre le tube de coulissement (13) et le tube support (15) suivi d'une étanchéification par introduction de soudure par capillarité entre les tubes de coulissement et support (13,15) et le manchon de centrage ou par surmoulage d'un cylindre d'étanchéification (64) en matière plastique.

3. Dispositif selon la revendication 1, **caractérisé en ce qu'**un dispositif d'obturation (67) est constitué d'un dispositif à bille dont la bille est appuyée sur un siège conique par un ressort et qui laisse passer le liquide de gonflage du dispositif d'alimentation (65) vers le ballonnet (25) mais empêche le liquide de gonflage de revenir.

4. Dispositif selon la revendication 1, **caractérisé en ce qu'**un deuxième dispositif interface de raccordement (66) est constitué d'un cône mâle venant s'emboîter dans un cône femelle constituant un premier dispositif interface de raccordement (32).

5. Dispositif selon la revendication 1, **caractérisé en ce qu'**un bouchon en caoutchouc (60) constitue un premier dispositif interface de raccordement et d'obturation coopérant avec un deuxième dispositif de raccordement constitué par une aiguille (61).

6. Dispositif selon la revendication 1, **caractérisé en ce qu'**un dispositif d'alimentation (65,68) est constitué d'une seringue composée d'un réservoir cylindrique dans lequel coulisse un piston dont la tige de commande est filetée et vissée dans un écrou solidaire du réservoir.

7. Dispositif selon la revendication 1, **caractérisé en ce qu'**une lame coupante (19,46) est de forme sensiblement triangulaire avec un premier côté de lame (20,48) fixé au tube de coulissement (13,39) suivant une de ses génératrices, un deuxième côté de lame (23,49), qui comporte un fil coupant, faisant un angle avec le premier côté de lame (20), dont le sommet coïncide sensiblement avec la première extrémité de tube de coulissement (18), de l'ordre de cinq à dix degrés.

8. Dispositif selon les revendications 1 et 7, **caractérisé en ce que** les lames coupantes (19,46) sont au nombre de quatre à six.

9. Dispositif selon la revendication 7, **caractérisé en ce qu'**un mode de fixation des lames coupantes (19) sur le tube de coulissement (13) consiste à faire une soudure au laser.

10. Dispositif selon la revendication 7, **caractérisé en ce qu'**un mode de fixation sur le tube de coulissement (39) des lames coupantes (46), comportant des perçages de lames (47) le long du premier côté de la lame (48), consiste à enserrer la base des lames coupantes (46) dans un tronc de cône de révolution (52), en matière plastique, enserrant aussi le tube de coulissement (39), les lames coupantes (46) étant verrouillées par rapport au tube de coulissement (39) par la matière plastique traversant les perçages de lames (47).

11. Dispositif selon la revendication 1, **caractérisé en ce qu'**un manche de manipulation (34) recouvre la partie du tube de coulissement (13) située au voisinage de la deuxième extrémité de tube de coulissement (33), la partie du tube support (15) et de la tubulure de remplissage (31) situées près de la deuxième extrémité de tube support (17).

## Claims

1. Device for positioning a guide tube (1) for a nephroscope, using a puncture needle (2), provided with a needle sleeve (7) and a closure mandrin (3), serving as a guide means for an expandable enlarging device (4, 36) fitted with a ballonet (25, 41) filled with an expansion liquid, **characterised in that** the expandable enlarging device (4, 36) is composed of a slide tube (13, 39) able to slide all along the puncture needle (2), of a support tube (15, 40), around which the ballonet (25, 41) is fixed, surrounding the slide tube (13, 39), of which the internal diameter is smaller than the internal diameter of the support tube (15, 40) forming a space for circulation of the expansion liquid communicating by a first orifice (29, 42) with the interior of the ballonet (25, 41) and by a second orifice with a filling tube (31, 57), provided with a closure device (67, 60) and connected to a first connection interface device (32, 60) able to cooperate with a second connection interface device (66, 61) communicating with a supply device (65, 68) for expansion liquid, the support tube (15, 40) being connected by at least one of its ends (16, 17, 44) to the slide tube (13, 39) in a sealed manner, a first slide tube end (18) of the slide tube (13) comprising at least two cutting blades (19, 46) forming a trocar and a second slide tube end (33) comprising a handling sleeve (34, 53).

2. Device as claimed in claim 1, **characterised in that** the sealing tightness between a first and second end (16, 17) of the support tube (13) and the slide tube (15) is achieved by necking or by positioning of a centring sleeve (43) between the slide tube (13) and the support tube (15) followed by creating a seal by introduction of capillary solder between the slide and support tubes (13, 15) and the centring sleeve or by overmoulding a sealing cylinder (64) made from synthetic material.

3. Device as claimed in claim 1, **characterised in that** a closure device (67) is formed by a ball device of which the ball is pressed onto a conical seat by a spring and which allows the expansion liquid to pass through from the supply device (65) to the ballonet (25) but prevents the expansion liquid from returning.

4. Device as claimed in claim 1, **characterised in that** a second connection interface device (66) is formed by a male cone coming to fit within a female cone forming a first connection interface device (32).

5. Device as claimed in claim 1, **characterised in that** a rubber plug (60) forms a first connection interface and closure device cooperating with a second connection device formed by a needle (61).

6. Device as claimed in claim 1, **characterised in that** a supply device (65, 68) is formed by a syringe composed of a cylindrical reservoir in which a piston slides, the control rod of which is threaded and screwed into a nut fixedly attached to the reservoir.

7. Device as claimed in claim 1, **characterised in that** a cutting blade (19, 46) is of a substantially triangular shape with a first blade side (20, 48) fixed to the slide tube (13, 39) along one of its generatrices, a second blade side (23, 49), which comprises a cutting thread, forming an angle with the first blade side (20), of which the tip coincides substantially with the first slide tube end (18), of the order of five to ten degrees.

8. Device as claimed in claims 1 and 7, **characterised in that** there are four to six cutting blades (19, 46).

9. Device as claimed in claim 7, **characterised in that** one manner of fixing the cutting blades (19) on the slide tube (13) consists of laser welding.

10. Device as claimed in claim 7, **characterised in that** one manner of fixing the cutting blades (46), comprising blade holes (47) along the first side of the blade (48), to the slide tube (39) consists of enclosing the base of the cutting blades (46) in a revolue truncated cone (52) of synthetic material, also enclosing the slide tube (39), the cutting blades (46) being locked with respect to the slide tube (39) by the synthetic material passing through the blade holes (47).

11. Device as claimed in claim 1, **characterised in that** a handling sleeve (34) covers the part of the slide tube (13) located in the proximity of the second slide tube end (33), the part of the support tube (15) and of the filling tube (31) which are located close to the second support tube end (17).

## Patentansprüche

1. Vorrichtung zum Anbringen einer Führungsröhre (1) für ein Nephroskop, das eine Punktionsnadel (2) verwendet, die mit einem Nadelstutzen (7) sowie mit einem Verschlussdorn (3) versehen ist, die als ein Führungsmittel für eine aufblasbare Erweiterungsvorrichtung (4, 36) dient, die mit einem Luftsack (25, 41) ausgestattet ist, der mit einer Aufblasflüssigkeit gefüllt ist, **dadurch gekennzeichnet, dass** die aufblasbare Erweiterungsvorrichtung (4, 36) aus einer Gleitröhre (13, 39) besteht, die ganz entlang der Punktionsnadel (2) gleiten kann, aus einer Tragröhre (15, 40), um welche der Luftsack (25, 41), der die Gleitröhre (13, 39) umgibt, befestigt ist, deren Innendurchmesser kleiner ist als der Innendurchmesser der Tragröhre (15, 40), die einen Zirkulationsraum der Aufblasflüssigkeit bildet, der über eine erste Öffnung (29, 42) mit dem Inneren des Luftsacks (25, 41) und über eine zweite Öffnung mit einem Füllrohrstutzen (31, 57) kommuniziert, der mit einer Verschlussvorrichtung (67, 60) versehen ist, die an eine erste Anschlussschnittstellenvorrichtung (32, 60) angeschlossen ist, die mit einer zweiten Anschlussschnittstellenvorrichtung (66, 61) zusammenwirken kann, die mit einer Vorrichtung (65, 68) zum Versorgen mit Aufblasflüssigkeit kommuniziert, wobei die Tragröhre (15, 40) über mindestens eines ihrer Enden (16, 17, 44) mit der Gleitröhre (13, 39) dicht verbunden ist, wobei ein erstes Gleitröhrenende (18) der Gleitröhre (13) mindestens zwei Schneidklingen (19, 46) aufweist, die einen Trokar bilden, und ein zweites Gleitröhrenende (33) einen Handhabungsgriff (34, 53) aufweist.

2. Vorrichtung nach Anspruch 1, **dadurch gekennzeichnet, dass** die Abdichtung zwischen einem ersten und einem zweiten Ende (16, 17) der Tragröhre (13) und der Gleitröhre (15) durch Einschnürung oder durch Positionieren eines Zentrierstutzen (43) zwischen der Gleitröhre (13) und der Tragröhre (15) gefolgt von einer Abdichtung durch Einführen durch Kapillaritätsschweißen zwischen der Gleitröhre (13) und der Tragröhre (15) und dem Zentrierstutzen oder durch Abformen eines Dichtzylinders (64) aus Kunststoff erzielt wird.

3. Vorrichtung nach Anspruch 1, **dadurch gekennzeichnet, dass** eine Verschlussvorrichtung (67) aus einer Vorrichtung mit Kugel besteht, deren Kugel auf einem kegelförmigen Sitz über eine Feder aufliegt und die Aufblasflüssigkeit der Versorgungsvorrichtung (65) zu dem Luftsack (25) gestattet, die Aufblasflüssigkeit jedoch daran hindert zurückzukommen.

4. Vorrichtung nach Anspruch 1, **dadurch gekennzeichnet, dass** eine zweite Anschlussschnittstellenvorrichtung (66) aus einem Einsetzkegel besteht, der in einen Hülsenkegel eingreift, der eine erste Anschlussschnittstellenvorrichtung (32) bildet.

5. Vorrichtung nach Anspruch 1, **dadurch gekennzeichnet, dass** ein Stopfen (60) aus Kautschuk eine erste Anschlussschnittstellen- und Verschlussvorrichtung bildet, die mit einer zweiten Anschlussvorrichtung, die aus einer Nadel (61) besteht, zusammenwirkt.

6. Vorrichtung nach Anspruch 1, **dadurch gekennzeichnet, dass** eine Versorgungsvorrichtung (65, 68) aus einer Spritze besteht, die aus einem zylindrischen Behälter besteht, in dem ein Kolben gleitet, dessen Bedienschaft ein Gewinde aufweist und in eine Mutter, die fest mit dem Behälter verbunden ist, eingeschraubt ist.

7. Vorrichtung nach Anspruch 1, **dadurch gekennzeichnet, dass** eine Schneidklinge (19, 46) eine im Wesentlichen dreieckige Form hat, mit einer ersten Klingenseite (20, 48), die an der Gleitröhre (13, 39) entlang einer ihrer Mantellinien befestigt ist, einer zweiten Klingenseite (23, 49), die einen Schneiddraht aufweist, der mit der ersten Klingenseite (20) einen Winkel in der Größenordnung von fünf bis zehn Grad bildet, dessen Scheitel im Wesentlichen mit dem ersten Ende der Gleitröhre (18) zusammenfällt.

8. Vorrichtung nach Anspruch 1 und 7, **dadurch gekennzeichnet, dass** es vier bis sechs Schneidklingen (19, 46) gibt.

9. Vorrichtung nach Anspruch 7, **dadurch gekennzeichnet, dass** eine Befestigungsart der Schneidklingen (19) an der Gleitröhre (13) darin besteht, eine Laserschweißung durchzuführen.

10. Vorrichtung nach Anspruch 7, **dadurch gekennzeichnet, dass** eine Befestigungsart an der Gleitröhre (39) der Schneidklingen (46) die Klingenbohrungen (47) entlang der ersten Seite der Klinge (48) aufweist, darin besteht, die Basis der Schneidklingen (46) in einen Rotationskegelstumpf (52) aus Kunststoff einzuschließen, der auch die Gleitröhre (39) einschließt, wobei die Schneidklingen (46) in Bezug zu der Gleitröhre (39) durch den Kunststoff, der die Klingenbohrungen (47) durchquert, verriegelt sind.

11. Vorrichtung nach Anspruch 1, **dadurch gekennzeichnet, dass** ein Handhabungsgriff (34) den Teil der Gleitröhre (13) abdeckt, der sich in der Nähe des zweiten Endes der Gleitröhre (33), des Teils der Tragröhre (15) und des Füllrohrstutzens (31) befindet, die sich in der Nähe des zweiten Endes der Tragröhre (17) befinden.
